Europäisches Patentamt
European Patent Office
Office européen des brevets

(19)

(11) Publication number: **0 282 309 A2**

(12)

# EUROPEAN PATENT APPLICATION

(21) Application number: 88302100.8

(22) Date of filing: 10.03.88

(51) Int. Cl.4: C 07 F 9/65
A 61 K 31/675

(30) Priority: 11.03.87 JP 56051/87
24.12.87 JP 327880/87

(43) Date of publication of application:
14.09.88 Bulletin 88/37

(84) Designated Contracting States:
AT BE CH DE ES FR GB GR IT LI LU NL SE

(71) Applicant: **YAMANOUCHI PHARMACEUTICAL CO. LTD.**
**No. 3-11 Nihonbashi-Honcho, 2-chome Chuo-ku**
**Tokyo (JP)**

(72) Inventor: **Sakamoto, Shuichi**
**3-17-7-402 Sakae-cho**
**Higashimurayama-shi Tokyo (JP)**

**Takeuchi, Makoto**
**Puregiru Enomoto 3-63-16-201 Higashi Oizumi**
**Nerima-ku Tokyo (JP)**

**Isomura, Yasuo**
**Tabata Sky Heights 4-12-11-919 Nishioku**
**Arakawa-ku Tokyo (JP)**

**Yoshida, Makoto**
**2-14-3 Nakahara-cho**
**Kawagoe-shi Saitama (JP)**

**Ohta, Mitsuaki**
**1-8-20, Minami**
**Wakou-shi Saitama (JP)**

**Abe, Tetsushi**
**7-8-9, Azuma-cho**
**Iruma-shi Saitama (JP)**

(74) Representative: **Geering, Keith Edwin et al**
**REDDIE & GROSE 16 Theobalds Road**
**London WC1X 8PL (GB)**

(54) **Azole-aminomethylene bisphosphonic acid derivatives.**

(57) Azole-aminomethylene bisphosphonic acids and lower alkyl esters and salts thereof, of the following general formula (I), are useful as drugs, e.g. as antiinflammatory and antirheumatic agents.

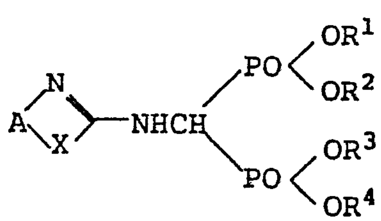

(I)

EP 0 282 309 A2

**Description**

AZOLE-AMINOMETHYLENE BISPHOSPHONIC ACID DERIVATIVES

This invention relates to azole-aminomethylene bisphosphonic acid derivatives useful as drugs, e.g. as antiinflammatory agents and antirheumatic agents.

As azolebisphosphonic acid derivatives, there have Unsubstituted nitrogen and sulfur-containing 5- and 6-membered heterocycle-thioalkyl bisphosphonic acids are described in European Patent Application No. 100,718A, and azole-alkyl bisphosphonic acids in U.S. Patent 4,687,767.

The present invention is concerned with azole-aminomethylene bisphosphonic acids in which the azole ring is bound to a methylene group via an imino group.

SUMMARY OF THE INVENTION

This invention provides azole-aminomethylene bisphosphonic acids, inclusive of lower alkyl esters and salts thereof, of formula (I) :

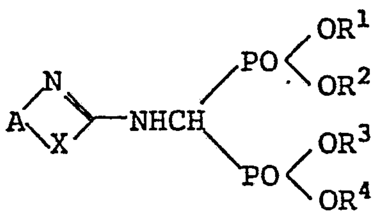 (I)

wherein A is

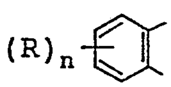 , 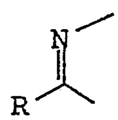 or 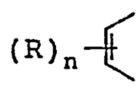

in which the or each R is selected from hydrogen and halogen atoms and lower alkyl and phenyl groups and n is 1 or 2; X is an oxygen or sulfur atom or an imino group; and $R^1$, $R^2$, $R^3$ and $R^4$ are selected independently from a hydrogen atom, lower alkyl groups, and salt moieties.

Preferred compounds of formula (I) include those wherein A is

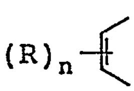

in which the or each R is selected from a hydrogen atom and lower alkyl groups; and X represents an oxygen or sulfur atom - e.g. [(5-methyl-2-oxazolyl)amino]methylene bis(phosphonic acid) and salts thereof.

In compounds (I), A forms a substituted or unsubstituted 5-membered heterocycle (azole) with the adjacent hetero atoms N and X. Typical examples of the heterocycle include thiazole, oxazole, imidazole, thiadiazole, oxadiazole, triazole, benzothiazole, and benzoxazole. The heterocycle may be substituted with at least one halogen atom and/or lower alkyl group. Suitable halogen atoms include fluorine, chlorine, bromine and iodine. Herein a lower alkyl group is a straight or branched hydrocarbon group containing 1 to 5 carbon atoms; Typical examples include methyl, ethyl, propyl, and isopropyl groups.

The Compound (I) salts are preferably nontoxic; the salts may be with bases including inorganic bases (e.g. sodium, potassium and ammonium salts) and organic bases(e.g. triethylamine salts).

The compounds of this invention can be produced by the following processes :

Process 1

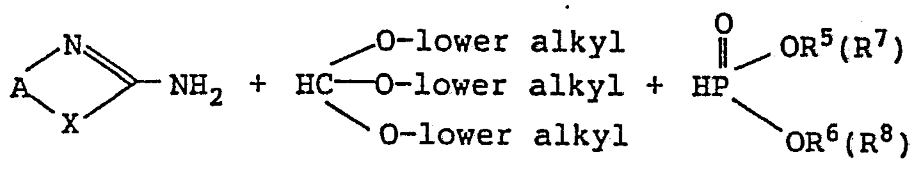

(II) (III) (IV)

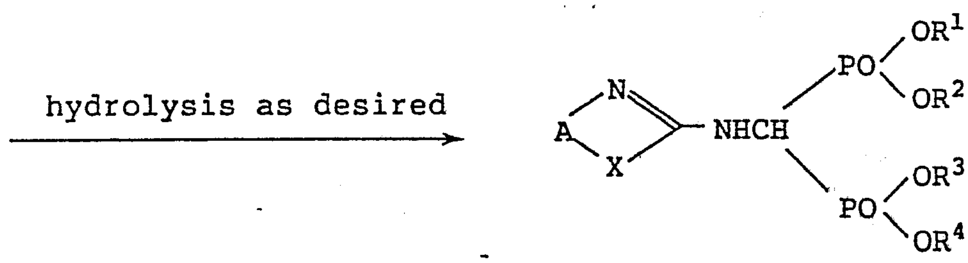


(I)

Process 2

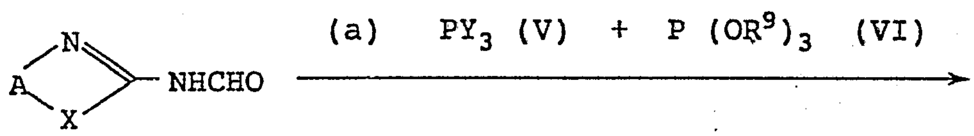


(VII)

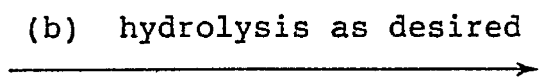


Compound (I)

In the above reaction formulae, A, X, $R^1$, $R^2$, $R^3$ and $R^4$ are as previously defined $R^5$, $R^6$, $R^7$, $R^8$ and $R^9$ are lower alkyl groups; and Y is a halogen atom.

In Process 1, the preferred compounds (II) are those wherein A is

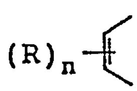

in which the or each R is selected from a hydrogen atom and lower alkyl groups; and X is an oxygen or sulfur atom. It is particularly preferred that compound (II) is 2-amino-5-methyloxazole with $R^5$, $R^6$, $R^7$ and $R^8$ being methyl or ethyl groups.

In Process 2, the preferred compounds (VII) are those wherein A is

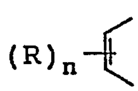

in which the or each R is selected from a hydrogen atom and lower alkyl groups; and X is an oxygen or sulfur atom. It is particularly preferred that compound (VII) is 5-methylthiazolyl-2-formamide and $R^9$ is methyl or ethyl.

Process 1

In this process 2-aminoazole (II), lower alkyl ($C_1$-$C_5$) orthoformate (III) and dialkyl phosphite (IV) may be mixed in stoichiometric proportions (or with excess dialkyl phosphite)and the reaction carried out with heating; a reaction solvent is not always necessary. The reaction is suitably conducted at 100 to 200°C, preferably at about 150°C, for 10 to 60 minutes.

The reaction product can be isolated and purified, for example by applying the reaction mixture to a silica gel column, followed by elution with a mixed solvent composed of methanol and chloroform.

Instead a reaction intermediate, imino ether of formula

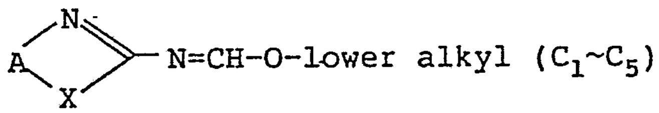

formed from 2-aminothiazole (II) and lower alkyl ($C_1$-$C_5$) orthoformate (III) may be isolated. Further reaction of this intermediate with phosphite (IV) gives the desired product (I).

The resulting bisphosphonate can be converted to the corresponding bisphosphonic acid by hydrolysis, if desired, e.g. by treatment with strong acid or with trimethylsilyl halide in a water-free solvent. Suitable hydrolysis reagents include hydrogen bromide in acetic acid (as commercially available or appropriately diluted), hydrogen halide gas-saturated solvent (e.g. chloroform, benzene, toluene) or a solution of iodotrimethylsilane (e.g. in carbon tetrachloride, di methylformamide, chloroform, toluene). The hydrolysis may be conducted with cooling or heating (preferably 0 to 50°C, more preferably about 25°C). Treatment with trimethylsilyl halide with cooling at -10°C or below leads to formation of the desired product in a partially hydrolyzed form. The hydrolysis may also be effected by heating under reflux in concentrated hydrochloric acid.

The bisphosphonic acid can be converted to salts in the usual manner by treatment with a base such as sodium hydroxide, potassium hydroxide or ammonia.

Process 2

In this process phosphorus trihalogenide (V) is first reacted with trialkyl phosphite (VI), e.g. at 40 to 100°C, preferably at 60 to 80°C, for 15 to 30 minutes. To the mixture is then added formylaminoazole (VII), and the resulting mixture is heated, for example at 40 to 100°C, preferably 60 to 80°C, for 1 to 8 hours. The progress of the reaction can be followed readily by TLC (developing system: chloroform-methanol). After completion of the reaction, the excess trialkyl phosphite is distilled off. The reaction product can be isolated and purified, for example, by applying the reaction mixture directly to a silica gel column or by dissolving it in chloroform, washing the chloroform solution with water, distilling off the solvent and then purifying on a silica gel column.

The resulting bisphosphonate may, as desired, be converted to the corresponding bisphosphonic acid, and further to a salt thereof, as described for Process 1.

Compounds provided by the present invention have bone absorption inhibiting effect and inhibitory effect on hypercalcemia resulting from bone absorption. Furthermore, they have antiinflammatory, antipyretic and analgesic activities.

The hypercalcemia inhibiting effect of compounds according to the invention have been demonstrated by the following test.

Hypercalcemia inhibiting effect

Typical effective ingredients according to the present invention were administered to parathyroid hormone (hereinafter referred to as "PTH")-induced hypercalcemia rats and, for each ingredient, the serum calcium level lowering effect was estimated.

Test method: Human 1 to 34 PTH (obtained from the Peptide Institute) was intravenously administered at a dose of 30 µg/kg to 5-week-old Wistar male rats fasted for 20 hours. For the administration, PTH was dissolved in physiological saline containing 0.1% BSA (bovine serum albumin) and the solution was given to the rats in an amount of 5 ml/kg. The normal control group was given 0.1% BSA-containing physiological saline alone in the same manner. Forty-five minutes after PHT administration, the rats were anesthetized with ether and then laparotomized, and blood samples were taken from the vena cava inferior using a vacuum blood-collecting tube. The blood samples were immediately centrifuged at 4°C, and 3,000 rpm (revolutions per minute) for 10 minutes. The thus-separated sera were measured for ionic calcium ($Ca^{++}$) concentration by means of a $Ca^{++}$ meter (Sera 250; Horiba Seisakusho).

The compounds according to the invention were dissolved, using sodium hydroxide and hydrochloric acid, in physiological saline (pH 7.4), for subcutaneous administration, in such amounts that the dose amounted to 2 ml/kg, and, for oral administration, in distilled water (pH 7.4) so that the dose amounted to 5 ml/kg. They were administered 24 or 72 hours before PTH administration. The normal control group and the control group were given physiological saline or distilled water in the same manner.

The results for each group were expressed in terms of mean ±S.E. (standard error) and comparison was made among the groups by testing by one-way analysis of variance. The significance level was taken at 5%.

The results thus obtained after subcutaneous administration and oral administration of the compounds

according to the invention are shown below in Table 1.

TABLE 1

| | Dose (mg/kg) | N | Serum $Ca^{++}$ (m moles/liter) |
|---|---|---|---|
| Subcutaneous administration | | | |
| Normal control | - | 5 | 1.48 ± 0.01** |
| Control | - | 5 | 1.63 ± 0.01 |
| Compound of Example 10 | 0.03 | 5 | 1.46 ± 0.01** |
| | 0.1 | 5 | 1.29 ± 0.01** |
| Oral administration | | | |
| Normal control | - | 5 | 1.42 ± 0.02 |
| Control | - | 5 | 1.48 ± 0.03 |
| Compound of Example 10 | 10 | 5 | 1.42 ± 0.03 |
| | 30 | 5 | 1.27 ± 0.03** |
| | 100 | 5 | 1.15 ± 0.04** |
| Subcutaneous administration | | | |
| Normal control | - | 5 | 1.41 ± 0.02* |
| Control | - | 5 | 1.52 ± 0.01 |
| Compound of Example 20 | 0.1 | 5 | 0.95 ± 0.01** |
| | 0.3 | 5 | 0.93 ± 0.03** |
| | 1.0 | 5 | 0.92 ± 0.03** |

TABLE 1 (cont'd)

| | Dose (mg/kg) | N | Serum $Ca^{++}$ (mmoles/liter) |
|---|---|---|---|
| Oral administration | | | |
| Normal control | - | 5 | 1.46 ± 0.01 |
| Control | - | 5 | 1.52 ± 0.02 |
| Compound of Example 20 | 10 | 5 | 1.39 ± 0.01* |
| | 30 | 5 | 1.25 ± 0.01** |
| | 100 | 5 | 1.12 ± 0.0 ** |
| Subcutaneous administration | | | |
| Normal control | - | 5 | 1.42 ± 0.02** |
| Control | - | 5 | 1.51 ± 0.02 |
| Compound of Example 21 | 0.1 | 5 | 1.48 ± 0.01 |
| | 0.3 | 5 | 1.40 ± 0.01** |
| | 1.0 | 5 | 1.30 ± 0.02** |
| Subcutaneous administration | | | |
| Normal control | - | 5 | 1.45 ± 0.03** |
| Control | - | 5 | 1.56 ± 0.01 |
| Compound of Example 6 | 0.1 | 5 | 1.49 ± 0.01 |
| | 0.3 | 5 | 1.45 ± 0.01** |
| | 1.0 | 5 | 1.41 ± 0.01** |
| Oral administration | | | |
| Normal control | - | 5 | 1.49 ± 0.02* |
| Control | - | 5 | 1.58 ± 0.03 |
| Compound of Example 6 | 10 | 5 | 1.58 ± 0.03 |
| | 30 | 5 | 1.54 ± 0.02 |
| | 100 | 5 | 1.45 ± 0.03** |

Notes to Table 1: Mean ± S.E., *: $p<0.05$, **: $p<0.01$.

The compound of Example 6 was administered 24 hours before PTH administration while other compounds were administered 72 hours before PTH administration.

The compounds according to the invention can be used not only as bone absorption inhibitors but also as antirheumatic agents, antiarthritic agents, antiinflammatory agents, antipyretics and analgesics.

The compounds according to the invention can be used as they are or in the form of pharmaceutical compositions prepared by admixing with pharmaceutically acceptable carrier or adjuvant or the like. Thus they may be administered orally in the form of tablets, capsules, powders, granules, pills and so forth or non-orally in the form of injections, syrups, ointments or suppositories, among others. The dose may vary depending on the subject, route of administration, symptoms and other factors but is generally within the range of 10 mg to 1 g per day for oral administration and 0.1 to 10 mg per day for parenteral administration, per human adult.

Dosage form examples containing a compound according to the invention are given below.

Tablets:

| | | |
|---|---|---|
| Compound of Example 10 | | 5 mg |
| Lactose | | 119 mg |
| Corn starch | | 67 mg |
| Hydroxypropylcellulose | | 4 mg |
| Carboxymethylcellulose calcium | | 4 mg |
| Magnesium stearate | | 1 mg |
| | Total | 200 mg |

The compound of Example 10 (0.5 g), 119 g of lactose and 67 g of corn starch are mixed together to give a homogeneous mixture. Then, 40 ml of 10% (w/w) aqueous solution of hydroxypropylcellulose was added, and the resulting mixture was wet-granulated. The granules thus obtained were mixed with 4 g of carboxymethylcellulose calcium and 1 g of magnesium stearate, and the whole mixture was tableted to give tablets each weighing 200 mg. Capsules:

| | | |
|---|---|---|
| Compound of Example 10 | | 5 mg |
| Crystalline cellulose | | 50 mg |
| Crystalline lactose | | 144 mg |
| Magnesium stearate | | 1 mg |
| | Total | 200 mg |

The above ingredients were mixed together in amounts 1,000 times those indicated above and the resulting mixture was filled, in 200-mg portions, into gelatin capsules to give capsule preparations.

Compounds according to the invention and methods of production thereof are described in greater detail in the following Examples which are for illustrative purposes only, and are not intended to limit the scope of the invention.

EXAMPLE 1

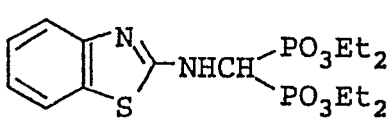

A mixture of 7.5 g of 2-aminobenzothiazole, 8.6 g of ethyl orthoformate and 14 g of diethyl phosphite was

heated at 150° C with stirring for 30 minutes. The reaction mixture was cooled to room temperature and the product was purified on a silica gel column (eluent; 2% methanol-chloroform) to give 4 g of tetraethyl [(2-benzothiazolyl)amino]methylene bis(phosphonate) as a solid.

The physico-chemical characteristics of this product are as follows:

(i) Mass spectrometric analysis (FAB Mass):

437 (M+1)

(ii) Nuclear magnetic resonance spectrum (in $CDCl_3$):

δ 1.2-1.4 (12H, $CH_3CH_2O$x4),
4.0-4.4 (8H, $CH_3CH_2O$x4),
5.28 (1H, dt, NHCH),
7.0-7.7 (4H, benzothiazole ring Hs)

EXAMPLE 2

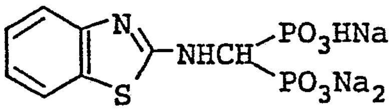

a) Iodotrimethylsilane (4.52 ml) was added dropwise to an ice-cooled solution of 3.3 g of tetraethyl [(2-benzothiazolyl)amino]methylene bis(phosphonate) in 30 ml of carbon tetrachloride. Then the temperature was allowed to rise to room temperature, and the mixture was stirred for 1 hours. The reaction mixture was concentrated, methanol was then added, and the mixture was again concentrated. The solid thus obtained was washed with chloroform.

b) This solid was dissolved in 0.1 N aqueous sodium hydroxide, and the solution was subjected to purification on an HP-20 resin column (eluent: water) to give 1.2 g of trisodium [(2-benzothiazolyl)amino-]methylene bis(phosphonate) as a hygroscopic solid.

The physico-chemical characteristics of this product are as follows:

(i) Elemental analysis ($C_8H_7N_2O_6SP_2Na_3 \cdot 0.5H_2O$)

| | C (%) | H (%) | N (%) |
|---|---|---|---|
| Calculated: | 24.07 | 2.02 | 7.02 |
| Found: | 24.10 | 2.46 | 7.02 |

(ii) Mass spectrometric analysis (FAB Mass):

391 (M+1)

(iii) Nuclear magnetic resonance spectrum (in $D_2O$):

δ 3.96 (1H, t, NHCH)
6.9-7.6 (4H, benzothiazole ring Hs)

EXAMPLE 3

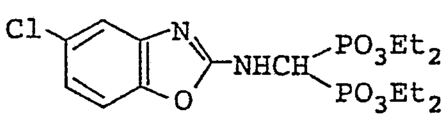

A mixture of 4.2 g of 2-amino-5-chlorobenzoxazole, 3.7 g of ethyl orthoformate and 6.9 g of diethyl phosphite was heated at 150° C with stirring for 30 minutes. The temperature was allowed to fall to room temperature, and the reaction product was purified in the same manner as in Example 1 to give 5 g of tetraethyl [(5-chloro-2-benzoxazolyl)amino]methylene bis(phosphonate) as a liquid.

The physico-chemical characteristics of this product are as follows:

(i) Mass spectrometric analysis (FAB Mass):

455, 457

(ii) Nuclear magnetic resonance spectrum (in $CDCl_3$):
δ 1.2-1.4 (12H, $CH_3CH_2Ox4$)
4.0-4.4 (8H, $CH_3CH_2Ox4$)
4.82 (1H, t, NHCH)
6.95-7.4(3H, benzoxazole ring Hs)

EXAMPLE 4

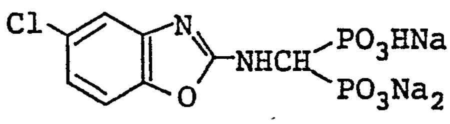

Iodotrimethylsilane (6.9 ml) was added to an ice-cooled solution of 5 g of tetraethyl [(5-chloro-2-benzoxazolyl)amino]methylene bis(phosphonate) in 25 ml of carbon tetrachloride. The reaction mixture was treated in the same manner as in Example 2. After purification, there are obtained 2.9 g of trisodium [(5-chloro-2-benzoxazolyl)amino]methylene bis)phosphonate) as a hygroscopic solid.

The physico-chemical characteristics of this product are as follows:

(i) Elemental analysis ($C_8H_6N_2O_7ClP_2Na_3 \cdot 1.5H_2O$)

| | C (%) | H (%) | N (%) |
|---|---|---|---|
| Calculated: | 22.06 | 2.08 | 6.43 |
| Found: | 22.07 | 2.18 | 6.18 |

(ii) Mass spectrometric analysis (FAB Mass):
409 (M+1)
(iii) Nuclear magnetic resonance spectrum (in $D_2O$):
δ 4.08 (1H, t, NHCH)
7.0-7.4 (3H, benzoxazole ring Hs)

EXAMPLE 5

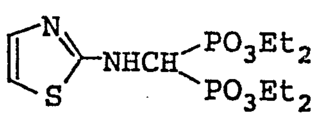

A mixture of 3 g of 2-aminothiazole, 5.3 g of ethyl orthoformate and 8.2 g of diethyl phosphite was heated at 150°C with stirring for 1 hour. The temperature was allowed to fall to room temperature, and the reaction product was purified in the same manner as in Example 1 to give 3.7 g of tetraethyl [(2-thiazolyl)amino-]methylene bis(phosphonate) as a solid.

The physico-chemical characteristics of this product are as follows:
(i) Mass spectrometric analysis (FAB Mass):
387 (M+1)
(ii) Nuclear magnetic resonance spectrum (in $CDCl_3$):
δ 1.2-1.3 (12H, $CH_3CH_2Ox4$)
4.0-4.4 (8H, $CH_3CH_2Ox4$)
5.08 (1H, t, NHCH)
6.55, 7.15 (each 1H, d, thiazole ring H)

EXAMPLE 6

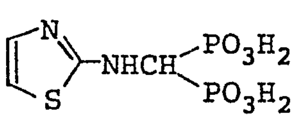

Iodotrimethylsilane (5.8 ml) was added to a solution of 3.7 g of tetraethyl [(2-thiazolyl)amino]meth ylene bis(phosphonate) in 74 ml of carbon tetrachloride with ice cooling. The subsequent treatment and purification procedures performed in the same manner as in Example 2 a) gave 1.3 g of [(2-thiazolyl)amino]methylenebis(phosphonic acid) as a hygroscopic acid.

The physico-chemical characteristics of this product are as follows:

(i) Elemental analysis ($C_4H_8N_2O_6SP_2 \cdot 1/3H_2O$)

| | C (%) | H (%) | N (%) |
|---|---|---|---|
| Calculated: | 17.14 | 3.10 | 10.00 |
| Found: | 17.33 | 3.10 | 9.87 |

(ii) Mass spectrometric analysis (FAB Mass):
275 (M+1)
(iii) Nuclear magnetic resonance spectrum (in $D_2O$):
δ 4.08 (1H, t, NHCH)
6.90, 7.22 (each 1H, d, thiazole ring H)

EXAMPLE 7

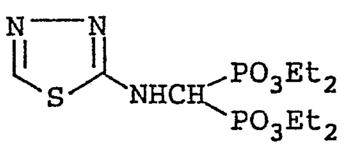

A mixture of 5 g of 2-amino-1,3,4-thiadiazole,7.3 g of ethyl orthoformate and 13.6 g of diethyl phosphite was heated at 150°C with stirring for 30 minutes. After cooling to room temperature, the reaction product was purified in the same manner as in Example 1 to give 3.2 g of tetraethyl [(1,3,4-thiadiazol-2-yl)amino]methylene bis(phosphonate) as a solid.

The physico-chemical characteristics of this product are as follows:
(i) Mass spectrometric analysis (FAB Mass):
388 (M+1)
(ii) Nuclear magnetic resonance spectrum (in DMSO-$d_6$):
δ 1.0-1.4 (12H, $CH_3CH_3O$x4)
3.9-4.3 (8H, $CH_3CH_2O$x4)
5.02 (1H, dt, NHCH)
8.42 (1H, d, NHCH)
8.68 (1H, s, thiadiazole ring H)

EXAMPLE 8

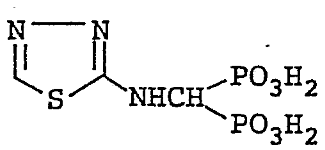

Iodotrimethylsilane (5 ml) was added to an ice-cooled solution of 3.2 g of tetraethyl [(1,3,4-thiadiazol-2-yl)amino]methylene bis(phosphonate) in 60 ml of carbon tetrachloride. The subsequent treatment and purification procedures performed in the same manner as in Example 2 a) gave 1.3 g of [(1,3,4-thiadiazol-2-yl)amino]methylene bis(phosphonic acid) as a hygroscopic solid.

The physico-chemical characteristics of this product are as follows:

(i) Elemental analysis ($C_3H_7N_3O_6SP_2 \cdot 1/3H_2O$)

| | C (%) | H (%) | N (%) |
|---|---|---|---|
| Calculated: | 12.81 | 2.49 | 14.94 |
| Found: | 13.01 | 2.31 | 15.21 |

(ii) Mass spectrometric analysis (FAB Mass):
276 (M+1)
(iii) Nuclear magnetic resonance spectrum (in $D_2O$):
δ 4.24 (1H, t, NHC<u>H</u>)
8.78 (1H, s, thiadiazole ring H)

EXAMPLE 9

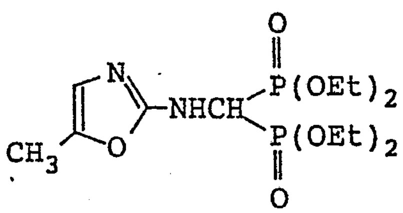

A mixture of 3.00 g (30.6 mmol) of 2-amino-5-methyloxazole [synthesized by the method described in Chem. Ber., 95, 2419 (1962)], 5.44 g (36.8 mmol) of ethyl orthoformate and 16.9 (122 mmol) of diethyl phosphite was heated on an oil bath at 145°C for 2.5 hours and then concentrated under reduced pressure (about 30 mmHg). The residue was subjected to two column chromatographic treatments (first: silica gel, chloroform-methanol; second: silica gel, ethyl acetate-acetone) to give 5.29 g (13.8 mmol) of tetraethyl[(5-methyl-2-oxazolyl)amino-]methylene bis(phosphonate) as an oil.

(i) Mass spectrometric analysis (EI):
384 ($M^+$), 247 (100), 109
(ii) Nuclear magnetic resonance spectrum (in $CDCl_3$):
δ 1.3 (bt, 12H), 2.2 (d, 3H), 4.0-4.2 (m, 8H), 4.6 (bt, 1H), 6.4 (q, 1H)

EXAMPLE 10

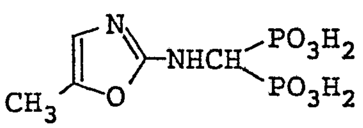

Tetraethyl [(5-methyl-2-oxazolyl)amino]methylene bis(phosphonate) (11 g) was dissolved in 11 ml of carbon tetrachloride, and 2.4 g of iodotrimethylsilane was added dropwise in an argon atmosphere with ice cooling. The temperature was then allowed to rise to room temperature, and the mixture was stirred for 45 minutes and thereafter concentrated. Methanol was added to the residue, and the mixture was again concentrated. The thus-obtained syrup solidified upon addition of acetonitrile. The solid was washed with hot methanol to give 0.5 g of [(5-methyl-2-oxazolyl)amino]methylene bis(phosphonic acid).

(i) Melting point: 250°C (decomposition)
(ii) Mass spectrometric analysis (FAB):
273 (M+1)

(iii) Elemental analysis ($C_5H_{10}N_2O_7P_2 \cdot H_2O$)

| | C (%) | H (%) | N (%) | P (%) |
|---|---|---|---|---|
| Calculated: | 20.70 | 4.17 | 9.66 | 21.35 |
| Found: | 20.73 | 3.62 | 9.98 | 21.22 |

EXAMPLE 11

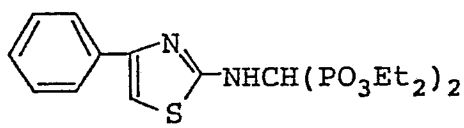

A mixture of 4.2 g of 2-amino-4-phenylthiazole, 4.2 g of ethyl orthoformate and 6.5 g of diethyl phosphite was heated at 160°C with stirring for 30 minutes. After cooling to room temperature, the reaction product was purified on a silica gel column (eluent: chloroform-methanol) to give 4 g of tetraethyl [(4-phenyl-2-thiazolyl)amino]methylene bis(phosphonate) as a solid.

(i) Mass spectrometric analysis (FAB Mass):
463 (M+1)

(ii) Nuclear magnetic resonance spectrum (in DMSO-$d_6$):
δ 1.04-1.36 (12H, $CH_3CH_2O$x4)
3.90-4.30 (8H, $CH_3CH_2O$x4)
5.22 (1H, dt, NHCH)
7.12 (1H, s, thiazole ring H)
7.36, 7.84, 8.36 (phenol ring Hs)

The following compounds were synthesized in the same manner as in Example 11:

EXAMPLE 12

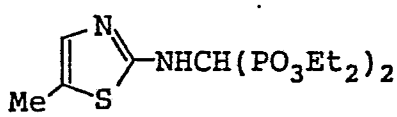

Tetraethyl [(5-methyl-2-thiazolyl)amino]methylene bis(phosphonate)

(i) Light-yellow oily substance

(ii) Mass spectrometric analysis (FAB Mass):
401 (M+1)

(iii) Nuclear magnetic resonance spectrum (in $CDCl_3$):
δ 1.2-1.4 (12H, $CH_3CH_2O$x4),

2.30 (3H, d, 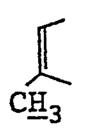 ),

4.0-4.4 (8H, $CH_3CH_2O$x4),
4.96 (1H, t, NHCH),

6.76 (1H, q, 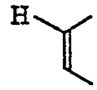)

EXAMPLE 13

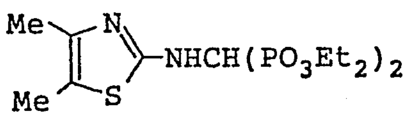

Tetraethyl [(4,5-dimethyl-2-thiazolyl)amino]methylene bis(phosphonate)
(i) Light-yellow oily substance
(ii) Mass spectrometric analysis (FAB Mass):
415 (M+1)
(iii) Nuclear magnetic resonance spectrum (in $CDCl_3$):
δ 1.2-1.4 (12H, $C\underline{H}_3CH_2O$x4),

2.08, 2.16 (each 3H, s, 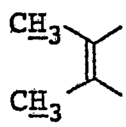),

4.0-4.4 (8H, $CH_3C\underline{H}_2O$x4),
4.90 (1H, t, NHC$\underline{H}$)

EXAMPLE 14

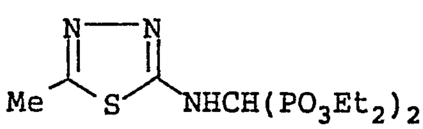

Tetraethyl [(5-methyl-1,3,4-thiazol-2-yl)amino]methylene bis(phosphonate)
(i) Light-yellow oily substance
(ii) Mass spectrometric analysis (FAB Mass):
402 (M+1)
(iii) Nuclear magnetic resonance spectrum (in $CDCl_3$):
δ 1.2-1.5 (12H, $C\underline{H}_3CH_2O$x4),

2.56 (3H, s, 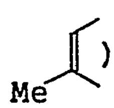),

3.96-4.40 (8H, $CH_3C\underline{H}_2O$x4),
5.04 (1H, t, NHC$\underline{H}$)

EXAMPLE 15

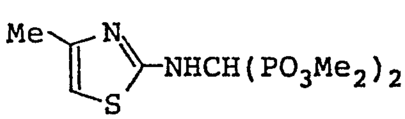

To a solution of 5.7 g of 2-amino-4-methylthiazole in 57 ml of dichloromethane was added dropwise with ice

cooling 14 ml of a formic acid-acetic acid mixture (5:3). Then the mixture was stirred overnight at room temperature. The reaction mixture was concentrated, and the solid obtained was washed with ether to give 6.6 g of 4-methylthiazolyl-2-formamide.

A mixture of 8.7 ml of trimethyl phosphite and 0.9 ml of phosphorus trichloride was heated at 65 to 70°C for 15 minutes. To this reaction mixture was added 1 g of 4-methylthiazolyl-2-formamide, and the mixture was stirred at that temperature for 1 hour. The reaction mixture was concentrated and subjected to purification on a silica gel column (eluent: chloroform-methanol) to give 1 g of tetramethyl [(4-methyl-2-thiazolyl)amino-]methylene bis(phosphonate) as a light-yellow oily substance.

The physico-chemical characteristics of this product are as follows:

(i) Mass spectrometric analysis (FAB Mass):
345 (M+1)

(iii) Nuclear magnetic resonance spectrum (in $CDCl_3$):

2.20 (3H, d, $C\underline{H}_3$ 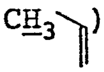),

3.8-4.0 (12H, $CH_3O$x4),
5.14 (1H, t, NHC$\underline{H}$),

6.10 (1H, q, 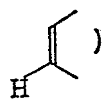)

The following compound was synthesized in the same manner as in Example 15:

EXAMPLE 16

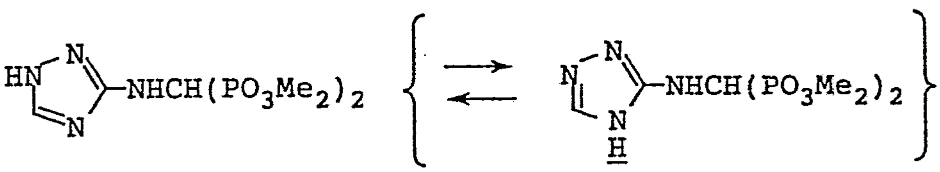

Tetramethyl [(1,2,4-triazol-3-yl)amino]methylene bis(phosphonate)

(i) Light-yellow oily substance

(ii) Mass spectrometric analysis (FAB Mass):
315 (M+1)

(iii) Nuclear magnetic resonance spectrum (in DMSO-$d_6$):
δ 3.6-3.8 (12H, $CH_3O$x4),
4.78 (1H, dt, NHC$\underline{H}$),

7.10 (1H, d, 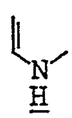),

7.8 (2H, N$\underline{H}$CH, 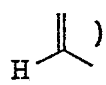)

The compounds of Examples 1, 3, 5, 7, 9, 11, 12, 13 and 14 can be produced in the same manner as in Example 15.

EXAMPLE 17

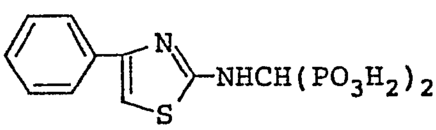

Iodotrimethylsilane (7 g) was added dropwise to an ice-cooled solution of 4 g of tetraethyl [(4-phenyl-2-thiazolyl)amino]methylene bis(phosphonate) in 40 ml of carbon tetrachloride. The temperature was allowed to fall to room temperature for one hour, and then the reaction mixture was concentrated. Methanol was added to the concentrate, and the mixture was again concentrated. The solid obtained was washed with methanol and then dissolved in 0.1 N aqueous sodium hydroxide, the pH being adjusted to 7. The solution was applied to an HP-20 resin column for purification (eluent: water) to give 0.6 g of trisodium [(4-phenyl-2-thiazolyl)amino]methylene bis(phosphonate).

The physico-chemical characteristics of this product are as follows:

(i) Elemental analysis ($C_{10}H_9N_2O_6SP_2Na_3 \cdot 0.5H_2O$)

| | C (%) | H (%) | N (%) |
|---|---|---|---|
| Calculated: | 28.23 | 2.35 | 6.59 |
| Found: | 28.45 | 2.91 | 6.64 |

(ii) Mass spectrometric analysis (FAB Mass):
417 (M+1)

(iii) Nuclear magnetic resonance spectrum (in $D_2O$):
δ 3.84 (1H, t, NHC<u>H</u>)
6.84 (1H, s, thiazole ring H)
7.4-7.8 (5H, phenyl ring Hs)

<u>EXAMPLE 18</u>

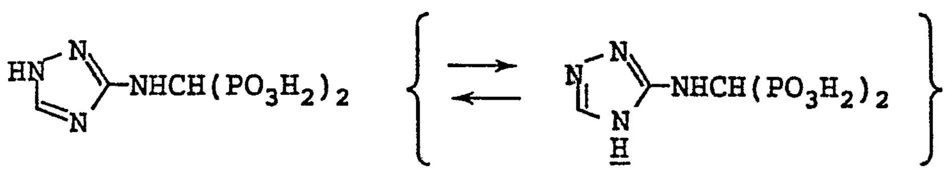

After the reaction was performed in the same manner as in Example 17, the product was purified without converting it to a sodium salt.

(1,2,4-Triazol-3-yl)aminomethylene bis(phosphoric acid).

(i) Elemental analysis ($C_3H_8N_4O_6P_2 \cdot 0.5H_2O$)

| | C (%) | H (%) | N (%) |
|---|---|---|---|
| Calculated: | 13.48 | 3.37 | 20.97 |
| Found: | 13.87 | 3.23 | 20.69 |

(ii) Mass spectrometric analysis (FAB Mass):
259 (M+1)

(iii) Nuclear magnetic resonance spectrum (in $D_2O$):
δ 4.12 (1H, t, NHC<u>H</u>),

8.22 (1H, s, 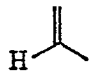 )

EXAMPLE 19

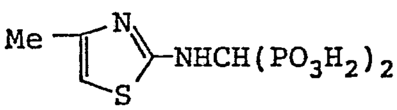

A solution of 600 mg of tetramethyl [(4-methyl-2-thiazolyl)amino]methylene bis(phosphonate) in 6 ml of concentrated hydrochloric acid was heated under reflux for 1.5 hours. The reaction mixture was concentrated. Water was added to the concentrate, whereupon crystals precipitated. Collection of the crystals by filtration gave 450 mg of [(4-methyl-2-thiazolyl)amino]methylene bis(phosphonic acid).

The physico-chemical characteristics of this product are as follows:

(i) Elemental analysis ($C_5H_{10}N_2O_6SP_2$)

| | C (%) | H (%) | N (%) |
|---|---|---|---|
| Calculated: | 20.84 | 3.50 | 9.72 |
| Found: | 20.57 | 3.50 | 9.52 |

(ii) Mass spectrometric analysis (FAB Mass):
289 (M+1)
(iii) Nuclear magnetic resonance spectrum (in $D_2O+K_2CO_3$):

δ 2.16 (3H, s, 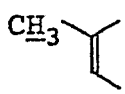 ),

3.72 (1H, t, NHC<u>H</u>)

6.18 (1H, d, 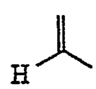 )

The following compounds were synthesized in the same manner as in Example 19:

EXAMPLE 20

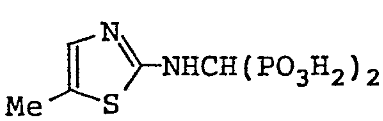

[(5-Methyl-2-thiazolyl)amino]methylene bis(phosphonic acid)

(i) Elemental analysis ($C_5H_{10}N_2O_6SP_2 \cdot 0.5H_2O$)

| | C (%) | H (%) | N (%) |
|---|---|---|---|
| Calculated: | 20.21 | 3.73 | 9.43 |
| Found: | 20.19 | 3.65 | 9.31 |

(ii) Mass spectrometric analysis (FAB Mass):
289 (M+1)
(iii) Nuclear magnetic resonance spectrum (in $D_2O+K_2CO_3$):

δ 2.26 (3H, s, 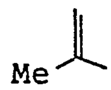 ),

3.85 (1H, t, NHC<u>H</u>)

6.80 (1H, d, 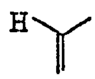 )

EXAMPLE 21

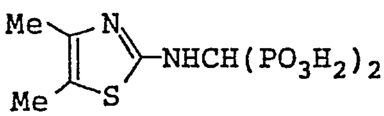

[(4,5-Dimethyl-2-thiazolyl)amino]methylene bis(phosphonic acid)

(i) Elemental analysis ($C_6H_{12}N_2O_6SP_2$)

| | C (%) | H (%) | N (%) |
|---|---|---|---|
| Calculated: | 23.85 | 4.00 | 9.27 |
| Found: | 23.36 | 3.94 | 9.07 |

(ii) Mass spectrometric analysis (FAB Mass):
303 (M+1)
(iii) Nuclear magnetic resonance spectrum (in $D_2O$):

δ 2.06, 2.16 (each 3H, s, 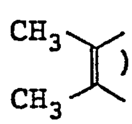 ),

3.60 (1H, t, NHC<u>H</u>)

EXAMPLE 22

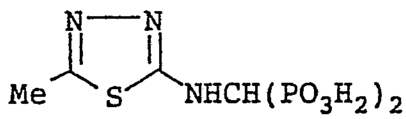

[(5-Methyl-1,3,4-thiadiazol-2-yl)amino]methylene bis(phosphonic acid)

(i) Elemental analysis ($C_4H_9N_3O_6SP_2$)

| | C (%) | H (%) | N (%) |
|---|---|---|---|
| Calculated: | 16.62 | 3.14 | 14.53 |
| Found: | 16.34 | 3.29 | 14.13 |

(ii) Mass spectrometric analysis (FAB Mass):
290 (M+1)
(iii) Nuclear magnetic resonance spectrum (in $D_2O$):

δ 2.62 (3H, s, 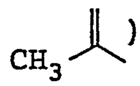 ),

4.20 (1H, t, NHC<u>H</u>)

**Claims**

1. An azole-aminomethylene bisphosphonic acid or lower alkyl ester or salt thereof of formula (I) :

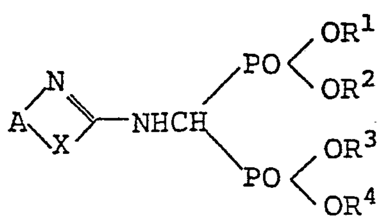 (I)

wherein A is

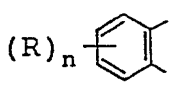 , 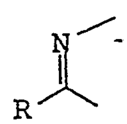 or 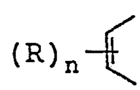

in which the or each R is selected from hydrogen and halogen atoms and $C_1$-$C_5$ alkyl and phenyl groups and n is 1 or 2; X is an oxygen or sulfur atom or an imino group; and $R^1$, $R^2$, $R^3$ and $R^4$ are selected independently from a hydrogen atom and $C_1$-$C_5$ alkyl groups.

2. A compound according to claim 1 wherein A is

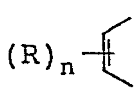

in which the or each R is selected from a hydrogen atom and $C_1$-$C_5$ alkyl groups; and X is an oxygen or sulfur atom.

3. A compound according to claim 1 which is [(5-methyl-2-oxazolyl)amino]-methylene bis(phosphonic acid) or an ester or salt thereof.

4. A pharmaceutical composition which comprises a compound according to claim 1, 2 or 3 and a

carrier therefore.

5. A method of producing an azole-aminomethylene bisphosphonic acid or a $C_1$-$C_5$ alkyl ester or salt thereof of formula (I)

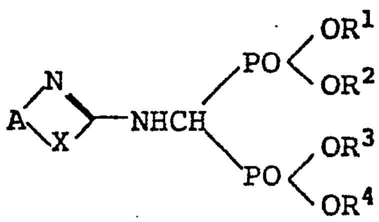

(I)

wherein A is

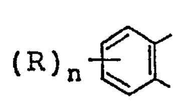 , 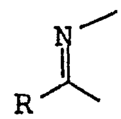 or 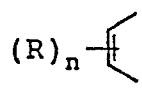

in which the or each R is selected from hydrogen and halogen atoms and $C_1$-$C_5$ alkyl and phenyl groups and n is 1 or 2; X is an oxygen or sulfur atom or an imino group; and $R^1$ $R^2$, $R^3$ and $R^4$ are selected independently from a hydrogen atom and $C_1$-$C_5$ alkyl groups which comprises reacting 2-aminoazole compound (II)

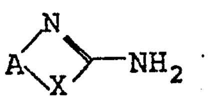

(II)

with ($C_1$-$C_5$ alkyl) orthoformate (III)

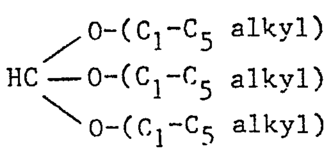

(III) and

and

dialkyl phosphite (IV)

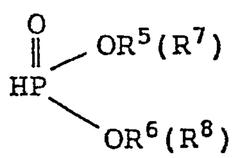

(IV)

wherein $R^5$, $R^6$, $R^7$ and $R^8$ are $C_1$-$C_5$ alkyl groups, and optionally hydrolyzing the resulting product and/or converting it to salt form.

6. A method according to claim 5 wherein A in formula (II) is

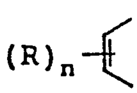

in which the or each R is selected from a hydrogen atom and $C_1$-$C_5$ alkyl groups; and X is an oxygen or sulfur atom.

7. A method according to claim 5 wherein compound (II) is 2-amino-5-methyloxazole and $R^5$, $R^6$, $R^7$ and $R^8$ are methyl or ethyl groups.

8. A method of producing an azole-aminomethylene bisphosphonic acid or a $C_1$-$C_5$ alkyl ester or salt thereof of formula (I)

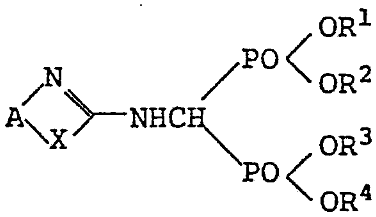

(I)

wherein A is

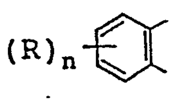 , 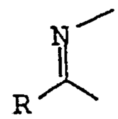 or 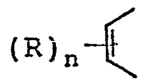

in which the or each R is selected from hydrogen and halogen atoms and $C_1$-$C_5$ alkyl and phenyl groups and n is 1 or 2; X is an oxygen or sulfur atom or an imino group; and $R^1$, $R^2$, $R^3$ and $R^4$ are selected independently from a hydrogen atom and $C_1$-$C_5$ alkyl groups which comprises reacting compound (VII)

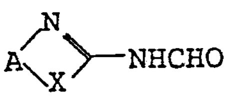

(VII)

with the reaction product of phosphorus trihalogenide (V)

$PY_3$ (V)

and trialkyl phosphite (VI)

$P(OR^9)_3$ (VI)

wherein Y is halogen and $R^9$ is $C_1$-$C_5$ alkyl, and optionally hydrolyzing the resulting product and/or converting it to salt form.

9. A method according to claim 8 wherein A in formula (VII) is

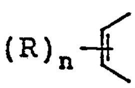

in which the or each R is selected from hydrogen atom and $C_1$-$C_5$ alkyl groups; and X is an oxygen or sulfur atom.

10. A method according to claim 8 wherein compound (VII) is 5-methylthiazolyl-2-formamide and $R^9$ is a methyl or ethyl group.